# EUROPÄISCHE PATENTSCHRIFT

(11) **EP 2 663 771 B1**
(45) Veröffentlichungstag und Bekanntmachung des Hinweises auf die Patenterteilung: **08.07.2015**
(21) Anmeldenummer: 13708180.8
(22) Anmeldetag: 08.03.2013
(51) Int. Cl.: F04B 7/00, F04B 13/00

(54) **KOLBENPUMPE**
PISTON PUMP
POMPE À PISTON

(30) Priorität: 19.03.2012 DE 102012102274
(43) Veröffentlichungstag der Anmeldung: 20.11.2013
(73) Patentinhaber: B. Braun Melsungen AG, 34212 Melsungen (DE)
(72) Erfinder: EBERHARD, Dietmar, 79341 Kenzingen (DE)
(74) Vertreter: Winter, Brandl, Fürniss, Hübner, Röss, Kaiser, Polte - Partnerschaft mbB
(86) Internationale Anmeldenummer: PCT/EP2013/054766
(87) Internationale Veröffentlichungsnummer: WO 2013/139629

(56) Entgegenhaltungen:
- DE-A1-102005 058 080
- DE-B3-102005 058 100
- DE-C1- 3 838 689
- DE-U1- 20 317 579
- US-B2- 7 887 308

## Beschreibung

Die Erfindung betrifft eine Kolbenpumpe zur Förderung eines Fluids, umfassend wenigstens einen Zylinder mit einem Kolben, der mittels eines Antriebs innerhalb des Zylinders entlang der Längsachse des Zylinders bewegbar ist, wobei jeder Zylinder an einer Stirnseite einen Montageflansch mit wenigstens einer Zylinderöffnung aufweist und sich zylinderseitig zwischen jedem Kolben und einem Montageflansch eine Kammer mit veränderlichem Volumen bildet, wenn der zugehörige Kolben im Zylinder bewegt wird. Ferner weist die Kolbenpumpe einen Einlassstutzen zur Zuführung und einen Auslassstutzen zur Abführung des Fluids auf.

Eine solche Kolbenpumpe eignet sich insbesondere für den Einsatz in der medizinischen Infusions- oder Dialysetechnik. Dort kommen derzeit vorwiegend Schlauch- und Spritzenpumpen zum Einsatz. Schlauchpumpen, die nach dem Peristaltikprinzip arbeiten, werden vor allem dann eingesetzt, wenn größere Flüssigkeitsmengen verabreicht werden sollen. Eine Bereitstellung dieser Flüssigkeitsmengen erfolgt beispielsweise durch Infusionsbeutel. Bei Spritzenpumpen ist das Fördervolumen durch den Spritzenkörper dagegen begrenzt und umfasst in der Regel nicht mehr als 50 ml, wobei einige Modelle auch einen Flüssigkeitsvorrat von bis zu 100 ml ermöglichen.

Schlauchpumpen nach dem Peristaltikprinzip sind sehr verbreitet und werden auch ambulatorisch beispielsweise für die künstliche Ernährung eingesetzt. Aufgrund ihres Pumpprinzips ist ihre Fördergenauigkeit schlechter als die der Spritzenpumpen. Periodisch arbeitende Kolbenpumpen hingegen weisen wie Spritzenpumpen eine hohe Fördergenauigkeit auf und können wie Peristaltikpumpen Fluide aus einem austauschbaren Vorratsbehälter ansaugen und weiterfördern.

Eine Kolbenpumpe für die medizinische Infusionstechnik ist beispielsweise in der US 7,887,308 B2 beschrieben. Diese Pumpe besitzt in einem ersten Ausführungsbeispiel ein zylindrisches Gehäuse, das radial gegenüber liegende Einlass- und Auslassstutzen aufweist. Innerhalb des Gehäuses bewegt sich ein Kolben durch einen daran angebrachten Rotor bi-direktional so, dass er sich axial entlang der Längsachse des Gehäuses bewegt und gleichzeitig auch um diese Längsachse dreht. Durch diese Bewegung werden abwechselnd Kanäle innerhalb des Kolbens jeweils mit einem Einlass- und Auslassstutzen verbunden, wodurch ein Fluid dosiert gefördert werden kann. In einem anderen Ausführungsbeispiel der Pumpe sind zwei koaxiale Kolben vorgesehen, die sich beide innerhalb eines beweglichen Gehäuses entlang der Längsachse dieses Gehäuses bewegen und so innerhalb des Gehäuses Kammern mit veränderlichem Volumen bilden. Zwischen den beiden Kolben ist eine Ventilscheibe angeordnet, in die gegenüber liegende Ein- und Auslassstutzen eingebracht sind. Die Ventilscheibe wird wie im ersten Ausführungsbeispiel durch einen daran angebrachten Rotor bi-direktional bewegt, wodurch sich das Gehäuse entlang der Längsachse der Kolben hin und her bewegt. Gleichzeitig werden durch die Drehung der Ventilscheibe um die Längsachse abwechselnd Kanäle innerhalb der Ventilscheibe freigegeben und die Zylinderkammern abwechselnd mit den Ein- und Auslassstutzen verbunden, wodurch die Pumpe kontinuierlich fördern kann. Da die Ein- und Auslassstutzen an der sich hin- und her bewegenden Ventilscheibe angebracht sind, bewegen sich die Verbindungsschläuche jedoch mit der Pumpbewegung, was zu erheblichen Bewegungen der beteiligten Pumpenteile führt und nachteilig sein kann. Insbesondere wird durch diese Funktionsweise viel Bauraum beansprucht, was unter anderem eine kompakte Bauweise der Pumpe erschwert.

Mittels einer periodisch arbeitenden Kolbenpumpe ist es somit möglich, auch bei großen Fördervolumen eine hohe Fördergenauigkeit zu erreichen. Allerdings ist es dabei oftmals erstrebenswert, eine solche Kolbenpumpe und andere Komponenten sehr kompakt auszubilden, um sie beispielsweise in ein möglichst kleines Gehäuse integrieren zu können. Dies ist zum Beispiel der Fall, wenn es sich um medizinische Einmalartikel und/oder Infusionstechnik im Bereich der häuslichen Pflege handelt, wobei Pumpen für austauschbare Infusionsbehälter einfach vom Patienten, ungelernten Angehörigen oder dem Pflegepersonal anzuschließen sein sollen. Hierbei hat es sich als vorteilhaft erwiesen, die gesamte Pumpe mit ihren zusätzlichen Komponenten in einer Art Kassette unterzubringen. Zu diesen zusätzlichen Komponenten zählen insbesondere Sensoriken zur Feststellung von Okklusionen und Luftblasen.

Die Erkennung einer Okklusion erfolgt meist durch eine indirekte Messung des Innendrucks in einem Schlauch, der zur Zuführung einer Flüssigkeit dient. Besteht eine Okklusion, erhöht sich beispielsweise stromab der Pumpe der Innendruck im Schlauch, was indirekt gemessen werden kann. Hierzu wird der runde Schlauchquerschnitt beispielsweise durch eine Vorspannkraft elliptisch verformt und der zu bestimmende Innendruck des Schlauchs erhöht oder vermindert diese Vorspannkraft, die dann mittels eines Kraftsensors ermittelt werden kann. Die DE 38 38 689 C1 offenbart exemplarisch ein derartiges Verfahren zur Druckmessung und Okklusionserkennung.

Nachteilig bei einer solchen Methode ist jedoch, dass die Verformung des Schlauchs in der Regel zu einem über Stunden anhaltenden Kriechvorgang führt. Dieses Kriechen baut Spannungen im Schlauchquerschnitt ab, was zu einer stetigen Änderung der gemessenen Kraft führt. Die durch das Kriechen verursachte und unerwünschte Kraftänderung ist in einer ähnlichen Größenordnung wie der gewünschte Messeffekt durch die Variation des Schlauchinnendrucks und erschwert somit die sichere Erkennung einer Okklusion. Spezielle Elastomere wie beispielsweise Silikone weisen ein deutlich vermindertes Kriechverhalten auf und sind daher als Schlauchsegment für die Okklusionssensorik prädestiniert. Eine Verbindung von Silikon- mit Nichtsilikonmaterialien ist allerdings sehr aufwändig, da prozesssichere Klebungen nicht möglich sind.

Ferner ist in medizinischen Infusions-Sets sowie bei Anwendungen in der Dialyse eine Luftblasenerkennung zwingend vorgeschrieben. Üblicherweise erfolgt die Erkennung über Ultraschallsender und -empfänger, die beidseitig schallmäßig an das zu observierende Schlauchstück angekoppelt werden. Allerdings ist es messtechnisch nicht möglich, ein Luftposter zwischen den Ultraschallkomponenten und einem fluidgefüllten Schlauch von einem luftgefüllten Schlauchstück zu unterscheiden. Daher wird üblicherweise das Schlauchstück durch spezielle Halterungen manuell zwischen die beiden Ultraschallkomponenten eingequetscht und entsprechend verformt, damit ein Luftpolster mit hoher Wahrscheinlichkeit vermieden wird.

Beim Einlegen eines Schlauchsets in eine peristaltische Pumpe müssen nach heutigem Stand der Technik daher oftmals die für die einzelnen Sensoriken zuständigen Schlauchsegmente per Hand in spezielle Halterungen eingelegt werden, was nicht nur im Bereich der häuslichen Pflege durchaus problematisch sein kann. Benachbarter Stand der Technik ist auch aus den Druckschriften US 2008 / 0 294 040 A1, US 2011 / 0 021 990 A1 und US 4 854 836 A bekannt. DE 20317 579 U1 ist ebenfalls einschlägig zu nennen.

Aufgabe der Erfindung ist es daher, eine Kolbenpumpe bereitzustellen, welche sowohl für die Pumpenfunktion als auch für zusätzliche Komponenten wie Okklusions- und Ultraschallsensoriken eine kompakte Bauweise und einfache Handhabung ermöglicht.

Erfindungsgemäß wird diese Aufgabe durch eine Kolbenpumpe gemäß dem unabhängigen Anspruch 1 gelöst. Vorteilhafte Weiterbildungen der Kolbenpumpe ergeben sich aus den Unteransprüchen 2-15.

Die erfindungsgemäße Kolbenpumpe dient zur Förderung eines Fluids und umfasst wenigstens einen Zylinder mit einem Kolben, der mittels eines Antriebs innerhalb des Zylinders entlang der Längsachse des Zylinders bewegbar ist, wobei jeder Zylinder an einer Stirnseite einen Montageflansch mit wenigstens einer Zylinderöffnung aufweist und sich zylinderseitig zwischen jedem Kolben und einem Montageflansch eine Kammer mit veränderlichem Volumen bildet, wenn der zugehörige Kolben im Zylinder bewegt wird. Ferner weist die Kolbenpumpe einen Einlassstutzen zur Zuführung und einen Auslassstutzen zur Abführung des Fluids auf.

Erfindungsgemäß ist auf der einem Zylinder abgewandten Seite eines Montageflansches eine drehbare Ventilscheibe angeordnet, die an dem jeweiligen Montageflansch anliegt. Dies bedeutet im Sinne dieser Erfindung, dass beispielsweise eine zylinderförmige Ventilscheibe mit einer Haupt- bzw. Deckfläche an dem Montageflansch anliegt. Dabei verläuft die Drehachse der Ventilscheibe vorzugsweise parallel zur Bewegungsrichtung des Kolbens, aber auch Abweichungen von der Parallelität sind von der Erfindung umfasst, so dass die Drehachse der Ventilscheibe auch lediglich in Richtung der Bewegungsrichtung des Kolbens verlaufen kann. Die Abweichung von der Parallelität kann beispielsweise in der Größenordnung von 1-20° liegen, ist jedoch nicht auf diese Werte beschränkt.

An wenigstens einem Montageflansch ist der Einlassstutzen und/oder Auslassstutzen angebracht, wobei ein Montageflansch im Bereich eines Einlassstutzens und eines Auslassstutzens jeweils wenigstens einen Durchlass aufweist, durch den das Fluid zwischen dem Einlassstutzen und/oder Auslassstutzen und der anderen Seite des Montageflansches strömen kann. Für die Ventilfunktion weist die Ventilscheibe Fluidüberführungsmittel auf, durch welche bei Drehung der Ventilscheibe zwischen zwei Winkelpositionen in beiden Winkelpositionen jeweils wenigstens eine Zylinderöffnung mit einem Durchlass eines Einlass- und/oder Auslassstutzens verbindbar ist.

Die Einlass- und Auslassstutzen sind somit unbeweglich und unabhängig von der Drehbewegung der Ventilscheibe. Hierdurch kann gegenüber anderen Pumpenkonzepten mit beweglichen Stutzen Bauraum eingespart werden. Ferner ist der Zusammenbau der Kolbenpumpe deutlich einfacher, und Verbindungen von Stutzen beispielsweise zu Schläuchen sind nicht durch die Bewegung der Stutzen gefährdet. Dabei ist die Drehbewegung der Ventilscheibe an die Hin- und Herbewegung der Kolben der Zylinder gekoppelt, so dass die Ventilfunktion kolbenstellungsabhängig ist.

In einem Ausführungsbeispiel der Erfindung verbinden die Fluidüberführungsmittel bei Drehung der Ventilscheibe in wenigstens einer dritten Winkelposition keine Zylinderöffnung mit einem Durchlass eines Einlass- und/oder Auslassstutzens. In einer solchen Winkelstellung können die Zylinder bzw. die Einlass- und/oder Auslassstutzens somit vollständig durch die Ventilscheibe verschlossen werden.

Vorzugsweise verläuft jeder Einlassstutzen und/oder Auslassstutzen in Richtung des wenigstens einen Zylinders. Dabei hat es sich als vorteilhaft erwiesen, dass jeder Einlassstutzen und/oder Auslassstutzen parallel zu dem wenigstens einen Zylinder verläuft, aber auch hier sind Abweichungen von der Parallelität von der Erfindung umfasst. Diese Abweichung kann erneut in der Größenordnung von 1-20° liegen, ist jedoch nicht auf diese Werte beschränkt. Somit kann auch die Drehachse der Ventilscheibe parallel oder zumindest in Richtung der Zylinderachse des wenigstens einen Zylinders verlaufen. Durch beide Maßnahmen lässt sich einzeln oder in Kombination eine besonders kompakte Bauweise der Kolbenpumpe erreichen.

Die Kolbenpumpe kann als Einkolbenpumpe ausgeführt sein, wobei sich sowohl ein einzelner Zylinder, als auch der Einlass- und der Auslassstutzen an einem gemeinsamen Montageflansch befinden. In einem weiteren Ausführungsbeispiel der Erfindung ist die Kolbenpumpe als Mehrkolbenpumpe ausgeführt, durch die sich eine unterbrechungsfreie Förderung erzielen lässt. Dazu sind beispielsweise zwei Montageflansche vorgesehen, zwischen denen sich die Ventilscheibe befindet. An beiden Montageflanschen ist jeweils ein Zylinder mit wenigstens einer Zylinderöffnung angebracht, während sich die Einlass- und Auslassstutzen gemeinsam an einem der beiden Montageflansche befinden. In einer anderen Ausführungsform einer solchen Mehrkolbenpumpe ist an jedem Montageflansch jeweils einer der Einlass- oder Auslassstutzen angebracht. An jedem Montageflansch befinden sich somit jeweils ein Zylinder und einer der Stutzen.

Die Fluidüberführungsmittel können auf verschiedene Arten ausgeführt sein, um eine Verbindung zwischen Zylinderöffnungen und Durchlässen zu den Stutzen zu realisieren. Beispielsweise können die Fluidüberführungsmittel wenigstens eine Vertiefung umfassen, die in eine einem Montageflansch mit Einlass- und/oder Auslassstutzen zugewandten Fläche der Ventilscheibe eingelassen ist, wobei die Vertiefung so ausgebildet ist, dass sie durch Drehung der Ventilscheibe gleichzeitig mit jeweils einer Zylinderöffnung und einem Durchlass eines Einlass- und/oder Auslassstutzens in Deckung bringbar ist. Ein derartiges Fluidüberführungsmittel dient zum flanschseitigen Fluidtransport auf einer Seite der Ventilscheibe und ist durch einen in die Ventilscheibe eingelassenen Kanal entsprechend ausgeformt.

Die Fluidüberführungsmittel können jedoch auch wenigstens eine Vertiefung umfassen, die ebenfalls in eine einem Montageflansch mit Einlass- und/oder Auslassstutzen zugewandten Fläche der Ventilscheibe eingelassen ist, jedoch ferner einen Durchlasskanal aufweist, der sich von dieser Vertiefung aus durch die Ventilscheibe hindurch erstreckt. Die Vertiefung und der zugehörige Durchlasskanal sind dann so ausgebildet, dass sie durch Drehung der Ventilscheibe gleichzeitig mit jeweils einer Zylinderöffnung und dem Durchlass eines gegenüber liegenden Einlass- und/oder Auslassstutzens in Deckung bringbar sind. Es handelt sich somit um ein Fluidüberführungsmittel, das einen Fluidtransport durch die Ventilscheibe hindurch ermöglicht.

Beide Varianten der Fluidüberführungsmittel können selektiv oder in Kombination eingesetzt werden, wobei die Auswahl im Wesentlichen von der gewählten Ausführungsform der Kolbenpumpe abhängt. Beispielsweise ist bei einer Einkolbenpumpe mit Einlass- und Auslassstutzen auf der Seite des Zylinders kein Fluidtransport durch die Ventilscheibe hindurch erforderlich, so dass als Fluidüberführungsmittel einfache Vertiefungen in einer Seite der Ventilscheibe ausreichen würden, um die Ventilfunktion zu erfüllen. Sobald Zylinder und/oder Einlass- und Auslassstutzen jedoch auf zwei Montageflansche verteilt werden, die gegenüber liegend auf den beiden Seiten der Ventilscheibe angeordnet sind, sind Fluidüberführungsmittel mit einem Durchlasskanal erforderlich, um einen Fluidtransport durch die Ventilscheibe hindurch zu ermöglichen.

Optional kann in einen Einlassstutzen und/oder in einen Auslassstutzen der Kolbenpumpe jeweils eine Okklusionssensorik integriert sein, die nicht zerstörungsfrei demontierbar ist. Beispielsweise kann dazu in dem Einlass- und/oder Auslassstutzen wenigstens eine Aussparung vorgesehen sein, welche dicht durch eine Sensorkomponente abgedeckt wird, die aus einem druckempfindlichen Material besteht. Dabei ist das Material des Einlass- und/oder Auslassstutzens härter als das der Sensorkomponente, und die Kolbenpumpe weist einen Kraftsensor auf, mit dem druckinduzierte Veränderungen der Sensorkomponente im Bereich der jeweiligen Aussparung messbar sind.

Die Erfindung kann sich somit das Funktionsprinzip einer Druckmembran zunutze machen, setzt dieses jedoch nicht in einem separaten Bauteil ein, sondern integriert eine entsprechende Sensorkomponente in einen Stutzen aus einem harten Material, durch den ein Fluid ohnehin gefördert wird. Dabei basiert die zu integrierende mechanische Sensorkomponente der Okklusionssensorik auf dem Prinzip der Druckmessung im Fluid und wird durch ein elastischen Material realisiert, das sich physikalisch analog einer Druckmembran verhält. Der Stutzen bildet eine druckunempfindliche Hartkomponente, die sich bei den auftretenden Druckänderungen nicht verformt. Aufgrund der auftretenden Verformungen der Sensorkomponente als Weichkomponente kann dagegen auf den Innendruck im Stutzen geschlossen werden.

Die Okklusionssensorik kann platzsparend direkt in einen harten Stutzen integriert werden, was eine sehr kompakte Bauweise ermöglicht. Bei dem Stutzen kann es sich um einen Einlass- und/oder Auslassstutzen handeln, der das Fluid zu einer Pumpe bzw. von dieser zu einem Patienten führt. Die Sensorik kann auf diese Weise Okklusionen vor und/oder hinter einer Pumpe erkennen. Wird der zugehörige Stutzen entsprechend so positioniert, dass er kompakt mit einer Pumpe in einem Gehäuse untergebracht werden kann, wird durch die Okklusionssensorik an diesem Stutzen kaum weiterer Bauraum benötigt.

Die Sensorkomponente ist dabei vorzugsweise ein integraler und nicht demontierbarer Bestandteil des Einlass- und/oder Auslassstutzens, so dass sie auch nicht bei Inbetriebnahme der Vorrichtung installiert oder sogar ausgerichtet werden muss. Dies erleichtert die Handhabung der Vorrichtung und vermeidet Einrichtungsfehler und somit auch Messfehler.

Vorzugsweise hat der Kraftsensor im Bereich der Aussparung Kontakt mit der Oberfläche der Sensorkomponente, wobei der Kraftsensor beispielsweise einen Stempel aufweist, der im Bereich der Aussparung in direktem Kontakt mit der Oberfläche der Sensorkomponente steht. So kann eine Veränderung der Ausdehnung der Sensorkomponente in diesem Bereich gemessen werden.

Ferner besteht die Sensorkomponente zweckmäßigerweise aus einem Elastomer, wobei es sich insbesondere um Silikon oder ein thermoelastisches Elastomer handeln kann. So können die physikalischen Eigenschaften dieser speziellen Elastomere vorteilhaft genutzt werden, was insbesondere ein geringes Kriechverhalten beinhaltet. Eine stoffschlüssige Verbindung von Silikon- und Nichtsilikonmaterialien ist dabei jedoch nicht erforderlich, da für eine dichte Verbindung zwischen Stutzen und Sensorkomponente geeignete Verfahren wie beispielsweise Spritzgussverfahren eingesetzt werden können. So können der Stutzen und die Sensorkomponente in einem Zweikomponentenverfahren hergestellt werden. Alternativ kann die Verbindung zwischen Stutzen und Sensorkomponente auch durch andere Verbindungstechniken hergestellt werden, wobei beispielsweise Steck-, Klick-, Schraub- oder auch Klebeverbindungen in Frage kommen.

In einem Ausführungsbeispiel der Okklusionssensorik ist die Sensorkomponente ein Schlauch, der den Stutzen formschlüssig umgibt, so dass sie die Aussparung dicht von außen abdeckt. In einem anderen Ausführungsbeispiel ist dieser Schlauch formschlüssig innerhalb des Stutzens angebracht, so dass die Sensorkomponente die Aussparung dicht von innen abdeckt. Dabei haben der Stutzen und die Sensorkomponente zweckmäßigerweise einen ähnlichen oder gleichen Querschnitt. Beispielsweise kann ein Schlauch mit rundem Querschnitt formschlüssig in einen runden Stutzen eingebracht werden bzw. diesen umschließen. Die Sensorkomponente muss jedoch nicht schlauchförmig sein, sondern kann jegliche Form haben, die für eine Abdeckung einer Aussparung geeignet ist.

Allerdings kann es auch vorteilhaft sein, wenn die Sensorkomponente einen elliptischen Querschnitt hat, wobei eine flache Seite der Sensorkomponente im Bereich der Aussparung angeordnet ist. Dies kann sowohl für innen als auch für außen liegende Sensorkomponenten der Fall sein, wobei der Querschnitt des Stutzens entsprechend angepasst werden muss. Durch diese Form der Weichkomponente hat die Sensorkomponente bereits die für die Innendruckmessung notwendige elliptische Verformung, so dass unerwünschtes Kriechverhalten bei thermoplastischen Elastomeren bereits weitestgehend unterbunden werden kann.

Der elliptische Querschnitt kann beispielsweise dadurch erreicht werden, dass ein Schlauch mit ursprünglich rundem Querschnitt entsprechend verformt wird, bevor er an einem Ein- oder Auslassstutzen montiert wird. Die Verformung wird dann nicht durch die Montage bewirkt, sondern um unerwünschtes Kriechverhalten zu unterbinden, erfolgt eine Vorverformung des Schlauches auf den gewünschten elliptischen Querschnitt.

In einem anderen Ausführungsbeispiel der Okklusionssensorik ist die Sensorkomponente eine speziell geformte Messmembran mit einem Querschnitt, der wenigstens zwei gegenüber liegende Membranseiten aufweist, die jeweils nach innen geknickt sind, während eine Membranoberseite, welche die beiden Membranseiten miteinander verbindet, gerade ausgeformt und im Bereich der Aussparung angeordnet ist. Der Kraftsensor liegt somit an einer geraden Fläche der Messmembran an, die nicht mehr durch die Eigenspannung verändert wird, so dass sich eine lineare Kraftkennlinie ergibt.

Weiterhin kann optional in einen Einlassstutzen und/oder in einem Auslassstutzen jeweils eine Ultraschallsensorik zur Erkennung von Luftblasen im jeweiligen Stutzen integriert sein, die nicht zerstörungsfrei demontierbar ist. In einem Ausführungsbeispiel der Erfindung ist die Ultraschallsensorik so ausgeführt, dass in den Einlass- und/oder Auslassstutzen formschlüssig ein Schlauch eingesteckt ist, durch den Fluid dem Einlassstutzen zugeführt oder durch den Fluid aus dem Auslassstutzen abgeführt wird. Dabei sind im Bereich des Schlauches an zwei Seiten des betreffenden Einlass- und/oder Auslassstutzens Flächen zur Ein- und Auskopplung von Ultraschall vorgesehen. Diese Flächen zur Ein- und Auskopplung von Ultraschall können plan ausgeführt sein, sie können jedoch auch anders geformte Oberfläche aufweisen, die an die Form der Ultraschallsensoren angepasst sind.

Der Einlass- und/oder Auslassstutzen ist vorzugsweise so ausgeformt, dass diese Flächen in einer Ebene liegen. Ferner kann es zweckmäßig sein, dass der Einlass- und/oder Auslassstutzen eine Aussparung aufweist, um eine mögliche Kurzschlussstrecke des Ultraschallwegs an dem zu untersuchenden Schlauchstück vorbei zu verhindern. In einem Ausführungsbeispiel der Erfindung liegt diese Aussparung gegenüber den Flächen zur Ein- und Auskopplung von Ultraschall, die Aussparung kann jedoch beliebig angeordnet sein. Auch mehrere Aussparungen für diesen Zweck sind möglich.

Mögliche Einsatzgebiete der erfindungsgemäßen Pumpe sind u.a. medizinische Einmalartikel von Infusions- oder Dialysesystemen oder Geräte mit Einmalartikeln zur individuellen Dosierung von Medikamenten z.B. im Apothekenbereich. Die beschriebene Anordnung kann in ein medizinisches Infusions-Set integriert werden und ersetzt dort das für die Förderung nötige Peristaltiksegment. In diese Kolbenpumpe können neben der reinen Pumpenfunktion mechanische Vorrichtungen integriert werden, die den mechanischen Teil der geforderten Fluidsensorik wie z.B. Okklusion- oder Luftblasenerkennung bilden und eine einfache mechanische Schnittstelle zu den externen elektronischen Sensorkomponenten darstellen. Bei Ausformung mit einer Sensorkomponente in einem Stutzen kann sie somit auch das für die Okklusionssensorik wichtige Druckmesssegment ersetzen. Durch die optionale Schnittstelle zu einem Ultraschallsensor, der potentielle Luftblasen erkennen kann, wird eine weitere Voraussetzung für ein kompaktes Kassettensystem für die Pump- und Sensoreinheit geschaffen.

Die mechanischen Sensorkomponenten müssen dabei nicht als eigenständige Komponenten durch aufwändige Montage oder Assemblierungstechniken in das Pumpenmodul eingesetzt werden, sondern können integrale und nicht demontierbare Bestandteile der Pumpe bilden. Hierzu wurden intelligente Anordnungen und ein Mechanikdesign gefunden, das kostengünstige Herstellungstechniken und insbesondere Mehrkomponenten-Spritzgusstechniken berücksichtigt. Die Pumpe selbst kann als periodisch arbeitende Kolbenpumpe realisiert werden, um die Vorteile der hohen Fördergenauigkeit sowie der Fördermöglichkeit aus einem Vorratsbeutel zu vereinen.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Von den Abbildungen zeigt:
- Fig. 1a: eine schematische Darstellung des Funktionsprinzips eines ersten Ausführungsbeispiels einer Kolbenpumpe als Einkolbenpumpe beim Ausstoßvorgang;
- Fig. 1b: eine Kolbenpumpe gemäß Fig. 1a beim Ansaugvorgang;
- Fig. 2a: eine schematische Darstellung des Funktionsprinzips eines zweiten Ausführungsbeispiels einer Kolbenpumpe als Mehrkolbenpumpe mit einseitigen Ein- und Auslassstutzen in einer ersten Kolbenstellung;
- Fig. 2b: eine Mehrkolbenpumpe gemäß Fig. 2a in einer zweiten Kolbenstellung;
- Fig. 3a: eine schematische Darstellung des Funktionsprinzips eines dritten Ausführungsbeispiels einer Kolbenpumpe als Mehrkolbenpumpe mit zweiseitigen Ein- und Auslassstutzen in einer ersten Kolbenstellung;
- Fig. 3b: eine Mehrkolbenpumpe gemäß Fig. 3a in einer zweiten Kolbenstellung;
- Fig. 4a: eine schematische Ansicht auf einen ersten Flansch mit einer Ventilscheibe für eine Mehrkolbenpumpe mit einseitigen Ein- und Auslassstutzen;
- Fig. 4b: eine schematische Ansicht auf einen zweiten Flansch mit einer Ventilscheibe für eine Mehrkolbenpumpe mit einseitigen Ein- und Auslassstutzen;
- Fig. 5a: den Funktionsmechanismus einer Ventilscheibe für eine Mehrkolbenpumpe mit einseitigen Ein- und Auslassstutzen für einen ersten Zylinder beim Ansaugvorgang;
- Fig. 5b: den Funktionsmechanismus einer Ventilscheibe für eine Mehrkolbenpumpe gemäß Fig. 5a für einen zweiten Zylinder beim Ausstoßvorgang;
- Fig. 6a: den Funktionsmechanismus einer Ventilscheibe gemäß Fig. 5a für den ersten Zylinder beim Ausstoßvorgang;
- Fig. 6b: den Funktionsmechanismus einer Ventilscheibe gemäß Fig. 5b für den zweiten Zylinder beim Ansaugvorgang;
- Fig. 7: einen Längsschnitt durch einen Stutzen mit außen liegender Sensorkomponente;
- Fig. 8: einen Querschnitt durch einen Stutzen gemäß Fig. 7;
- Fig. 9: einen Längsschnitt durch einen Stutzen mit einem ersten Ausführungsbeispiel einer innen liegenden Sensorkomponente;
- Fig. 10: einen ersten Querschnitt durch einen Stutzen gemäß Fig. 9;
- Fig. 11: einen zweiten Querschnitt durch einen Stutzen gemäß Fig. 9;
- Fig. 12: einen Querschnitt durch einen Stutzen mit einem zweiten Ausführungsbeispiel einer innen liegenden Sensorkomponente;
- Fig. 13: einen Längsschnitt durch einen Stutzen mit innen liegender Sensorkomponente und Ultraschallsensorik;
- Fig. 14: einen Querschnitt durch einen Stutzen mit Ultraschallsensorik gemäß Fig. 13;

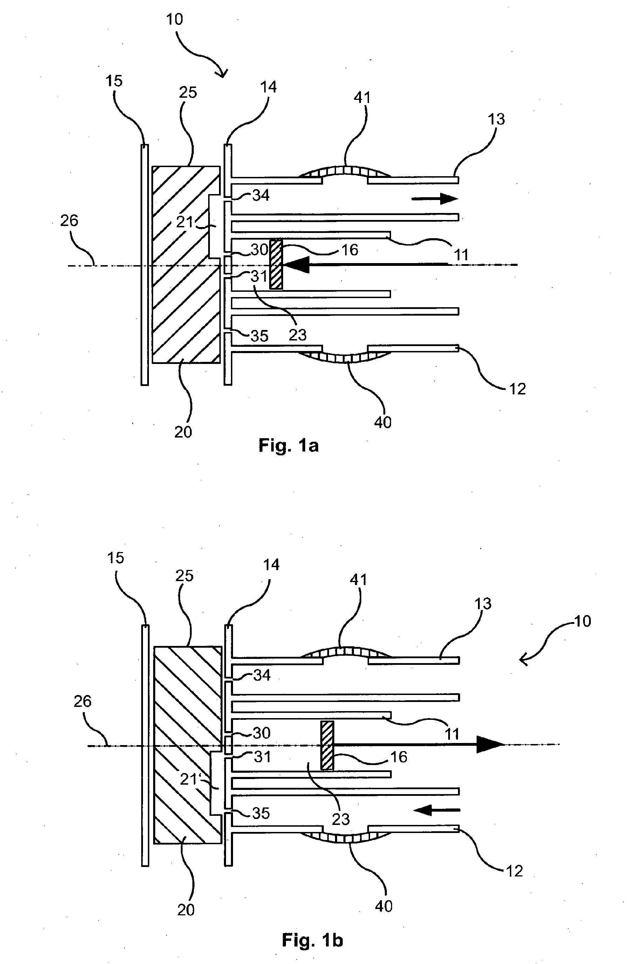

Fig. 1a und Fig. 1b zeigen jeweils in einer schematischen Darstellung ein Ausführungsbeispiel der erfindungsgemäßen Kolbenpumpe in einer Ausführungsform als Einkolbenpumpe. Dabei zeigt Fig. 1a die Einkolbenpumpe beim Ausstoßvorgang, während Fig. 1b den Ansaugvorgang zeigt. Die Bewegung eines Kolbens 16 und korrespondierend dazu eines zu fördernden Fluids ist für das Ansaugen und das Ausstoßen jeweils mit einem Pfeil gekennzeichnet.

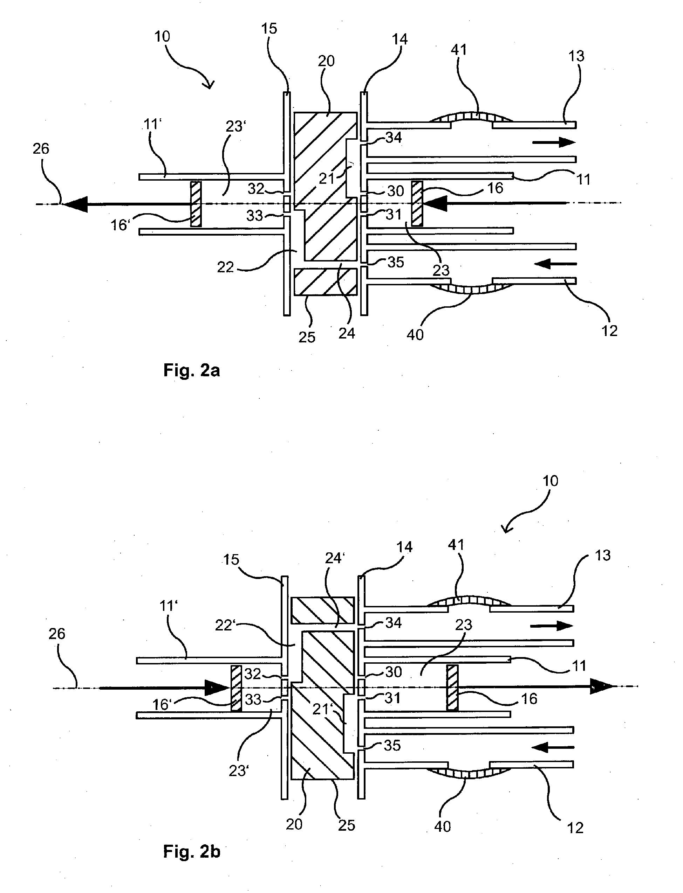

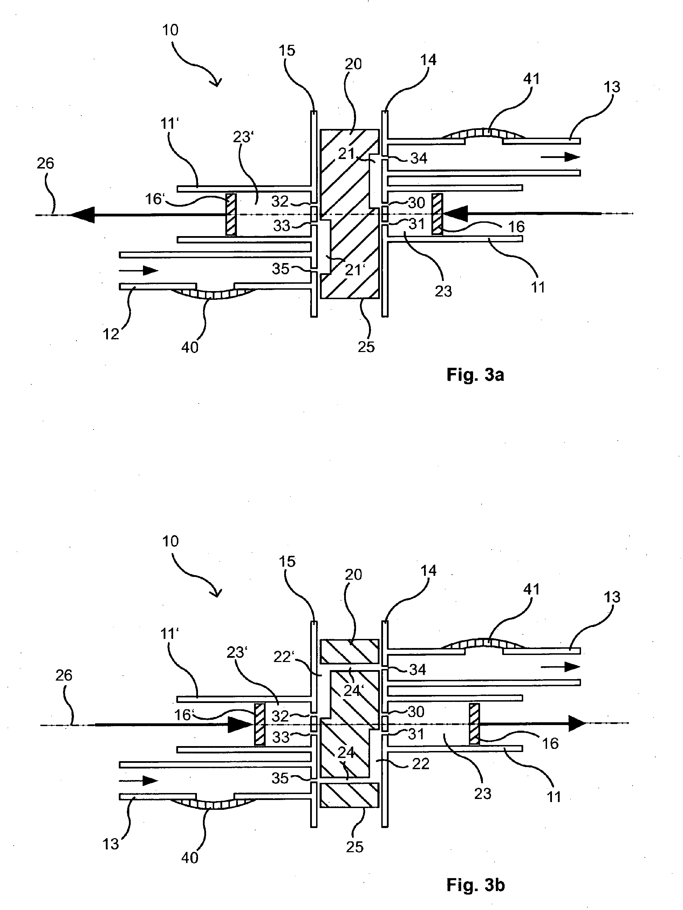

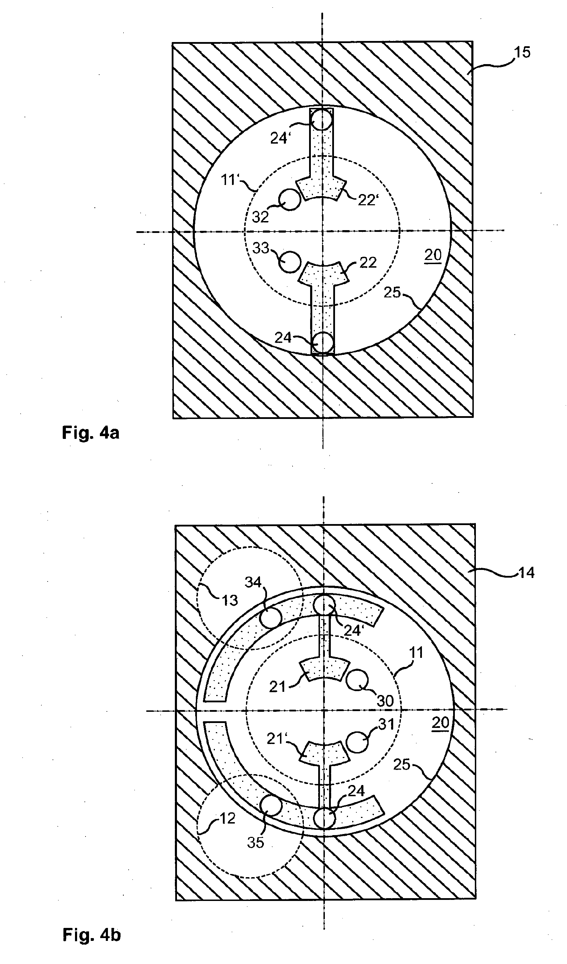

Dabei stellt die schematische Darstellung der Figuren 1 a und 1 b keinen geraden Schnitt durch die Kolbenpumpe dar, sondern zeigt lediglich den prinzipiellen Weg eines Fluids beim Pumpvorgang. Dargestellte Zylinder, Stutzen und Öffnungen sind somit nicht zwingend in einer Ebene geschnitten, sondern können auch in verschiedenen Ebenen liegen. Das Gleiche gilt für die Darstellungen der Figuren 2a, 2b, 3a und 3b. Ein Ausführungsbeispiel von Zylindern, Stutzen und Öffnungen in versetzten Ebenen ist beispielsweise aus der Ansicht der Figuren 4a und 4b ersichtlich.

Bei der Einkolbenpumpe 10 gemäß den Figuren 1a und 1b ist ein erster Flansch 14 mit einem,Zylinder 11 versehen, in den ein Kolben 16 beweglich eingebracht ist. Ein solcher Flansch 14 kann auch als Montageflansch bezeichnet werden, da er nicht nur an der Stirnseite den Umfang des Zylinders 11 umgibt, sondern weitere Komponenten an diesem Montageflansch angebracht sein können. Am Flansch 14 sind insbesondere ein Einlassstutzen 12 und ein Auslassstutzen 13 angebracht. Der Zylinder 11 liegt vorzugsweise zwischen dem Einlass- und dem Auslassstutzen 12, 13, und die Längsachsen des Zylinders 11 und der Einlass- und dem Auslassstutzen 12, 13 verlaufen im Wesentlichen parallel zueinander. Durch eine Bewegung des Kolbens 16 innerhalb des Zylinders 11, die durch einen nicht dargestellten Antrieb bewirkt wird, bildet sich zylinderseitig zwischen dem Flansch 14 und dem Kolben 16 eine Kammer 23 mit veränderlichem Volumen. Im Bereich der Stirnseite des Zylinders 11 ist im Flansch 14 wenigstens eine Zylinderöffnung vorgesehen, durch die Fluid zwischen dem Zylinder 11 und der anderen Seite des Flansches 14 strömen kann, wenn der Kolben 16 bewegt wird.

In den in den Figuren dargestellten Ausführungsformen sind für jeden Zylinder 11 jeweils zwei Zylinderöffnungen 30 und 31 vorgesehen. Ferner ist in jedem Stutzen 12, 13 jeweils ein Durchlass 34, 35 im Flansch 14 vorgesehen, durch den Fluid ebenfalls zwischen dem jeweiligen Stutzen und der anderen Seite des Flansches 14 strömen kann.

Die Umschaltung der Funktionen Ansaugen und Ausstoßen der Pumpe 10 wird über eine hin- und her rotierende Ventilscheibe 20 realisiert. Dabei dreht sich die Ventilscheibe 20 um die Drehachse 26 zwischen wenigstens zwei Winkelpositionen, wobei es vorteilhaft sein kann, wenn die Drehachse 26 der Ventilscheibe 20 mit der Zylinderachse und somit mit der Bewegungsrichtung des Kolbens 16 zusammenfällt, wie es in den Figuren der Fall ist. Dies ist aber keine Zwangsbedingung der Erfindung.

Die Ventilscheibe 20 grenzt an ihrer gegenüber liegenden Seite an einen weiteren Flansch, der mit 15 bezeichnet ist. Dabei liegt die Ventilscheibe 20 an beiden Flanschen 14, 15 an und weist Fluidüberführungsmittel auf, mit denen ein zu förderndes Fluid über die Ventilscheibe zwischen Zylindern und Stutzen bewegt werden kann. Vorzugsweise handelt es sich bei den Fluidüberführungsmitteln um wenigstens eine Vertiefung 21, 21', die in die Oberfläche der Ventilscheibe 20 eingelassen ist und somit bei Drehung der Ventilscheibe 20 ihre Lage verändert. Diese wenigstens eine Vertiefung realisiert die Ventilfunktion, wobei eine Vertiefung beispielsweise durch kreisförmige, gebogene oder gerade vertiefte Kanäle in der Oberfläche der Ventilscheibe 20 realisiert werden kann. Ein Dichtmedium an den Rändern der Vertiefung sorgt vorzugsweise dafür, dass das Fluid die Kanalgeometrie nicht verlassen kann. Hierzu können beispielsweise Dichtlippen eingesetzt werden, oder die Oberflächen der Ventilscheibe 20 bilden durch ein spezielles Material eine Dichtfläche aus.

Statt Vertiefungen 21, 21' in der Oberfläche der Ventilscheibe 20 können jedoch auch andere Formen von Kanälen zum Transport eines Fluids über bzw. durch die Ventilscheibe 20 vorgesehen werden. Beispielsweise kann es sich auch um Bohrungen durch die Ventilscheibe handeln, die flanschseitig unter der Oberfläche der Ventilscheibe verlaufen und dann durch ihre zwei Öffnungen auf dieser Seite den Transport eines Fluids durch die Ventilscheibe realisieren.

Beim Ausstoßvorgang der Fig. 1a ist eine Vertiefung 21 im oberen Bereich in Deckung sowohl mit einer Zylinderöffnung 30 als auch mit einem Durchlass 34 im Auslassstutzen 13. Die Zylinderöffnung 31 und der Durchlass 35 im Einlassstutzen 12 sind dagegen von der Ventilscheibe 20 verdeckt und somit verschlossen. Bei einer Bewegung des Kolbens 16 in Pfeilrichtung wird somit zuvor angesaugtes Fluid aus der Kammer 23 durch die Zylinderöffnung 30 in die Vertiefung 21 und von dort durch den Durchlass 34 in den Auslassstutzen 13 gedrückt. Von dort kann das Fluid beispielsweise über einen Schlauch weiteren Komponenten bzw. einem Patienten zugeführt werden.

Nach dem Ausstoßen dreht die Ventilscheibe 20 in eine zweite Winkelposition, die in Fig. 1b zu sehen ist und bei der sich nun eine Vertiefung 21' im unteren Bereich in Deckung mit der Zylinderöffnung 30 und gleichzeitig auch mit dem Durchlass 35 im Einlassstutzen 12 in. Bewegt sich der Kolben 16 gleichzeitig in Pfeilrichtung, wird Fluid durch den Einlassstutzen 12 aus einem nicht dargestellten Vorratsbehälter angesaugt. Dabei wird das Fluid aus dem Einlassstutzen 12 durch den Durchlass 35 in die Vertiefung 21' und von dort durch die Zylinderöffnung 30 in die Kammer 23 im Zylinder 11 gesaugt.

Beim Ausstoßen ist die Verbindung vom Zylinder 11 zum Auslassstutzen 13 daher durch einen bestimmten Winkelbereich der Ventilscheibe 20 freigeschaltet, während ein anderer Winkelbereich beim Ansaugen den Einlassstutzen 12 mit dem Zylinder 11 verbindet. Die Ventilsteuerung erfolgt somit durch eine kolbenhubabhängige Hin- und Herbewegung wenigstens eines Kolbens. Wie in den Figuren 1a und 1b zu sehen, ist eine Vertiefung 21, 21' der Ventilscheibe 20 dazu abwechselnd mit einer anderen der beiden Zylinderöffnungen 30, 31 in Deckung. Es kann jedoch auch vorgesehen sein, dass nur eine Zylinderöffnung vorgesehen ist, wobei die Vertiefungen 21, 21' und diese Zylinderöffnung entsprechend ausgeformt und positioniert sind, um in beiden Winkelpositionen der Ventilscheibe 20 in Deckung zu sein.

In dem dargestellten Ausführungsbeispiel der Figuren 1a und 1b sind zwei Vertiefungen 21 und 21' vorgesehen, die jedoch nicht symmetrisch auf der Ventilscheibe angeordnet sein müssen, sondern auch in einem Winkel von weniger als 180° versetzt zueinander positioniert sein können. Beispielsweise kann die Ventilfunktion durch zwei Vertiefungen erreicht werden, die 90° versetzt zueinander angeordnet sind. So muss die Ventilscheibe auch nur um diesen Winkel gedreht werden, um zwischen den beiden Stellungen zu wechseln. Je nach Anordnung der Stutzen 12, 13 und des Zylinders 11 an dem Flansch 14 lässt sich dieser Winkel weiter reduzieren oder vergrößern.

Die Ventilfunktion kann jedoch auch durch eine einzelne Vertiefung erreicht werden, die zwischen den beiden beschriebenen Winkelpositionen hin- und herbewegt wird. In diesem Fall wären die Vertiefungen 21 und 21' identisch. Dann müsste die Ventilscheibe 20 je nach Anordnung der Stutzen 12, 13 und des Zylinders 11 gegebenenfalls um einen größeren Winkelbereich gedreht werden, um die Vertiefung in die erforderlichen zwei Positionen zu bringen.

Aus der Einkolbenlösung können weitere Varianten mit mehreren Zylindern abgeleitet, die beispielhaft in den Figuren 2a bis 3b dargestellt sind. Entsprechend den Figuren 2a und 2b wird beispielsweise ein zweiter Zylinder 11' mit Kolben 16' in den Flansch 15 integriert. Beide Kolben 16, 16' bewegen sich stets in gleicher horizontaler Richtung, wodurch ein Kolben je nach Stellung der Ventilscheibe 20 die Ansaugfunktion und der andere das Ausstoßen übernimmt.

Beim Ausstoßen durch den Kolben 16 wird zuvor angesaugtes Fluid durch die Zylinderöffnung 30 in die Vertiefung 21 und von dort durch den Durchlass 34 in den Auslassstutzen 13 gedrückt. Gleichzeitig wird durch die Bewegung des anderen Kolbens 16' Fluid aus dem Einlassstutzen 12 durch den Durchlass 35 in einen Kanal 24 innerhalb der Ventilscheibe 20 gesaugt. Dieser Kanal 24 verläuft durch die Ventilscheibe 20 hindurch und verbindet beide Seiten miteinander. Der Kanal 24 mündet in eine weitere Vertiefung 22 auf der anderen Seite der Ventilscheibe 20, welche so ausgeformt und angeordnet ist, dass sie mit der Zylinderöffnung 33 im Zylinder 11' in Deckung ist. Auf diesem Weg kann das Fluid von dem Einlassstutzen 12 in die Kammer 23' des Zylinders 11' strömen.

Bewegen sich die Kolben 16, 16' nun, wie in Fig. 2b dargestellt, in die andere Richtung, befindet sich die Ventilscheibe 20 in einer zweiten Winkelposition. Nun wird Fluid aus dem Einlassstutzen 12 durch den Durchlass 35, die Vertiefung 21' und die Zylinderöffnung 31 in die Kammer 23 gesaugt, während das zuvor in die Kammer 23' gesaugte Fluid durch den Durchlass 32, die Vertiefung 22', den Kanal 24' und den Durchlass 34 auf die andere Seite der Ventilscheibe 20 in den Auslassstutzen 13 gedrückt wird. Durch Wiederholung dieser Vorgänge wird die Förderung nahezu unterbrechungsfrei.

Es sind somit unterschiedliche Arten von Vertiefungen 21 und 22 in der Ventilscheibe vorgesehen, wobei eine erste Vertiefungsart 21 dazu dient, Fluid auf einer Seite der Ventilscheibe zwischen einem Zylinder und einem Stutzen zu transportieren. Diese Art von Vertiefung ist durch entsprechende Kanäle in der Oberfläche der Ventilscheibe 20 ausgeformt und stellt eine Vertiefung für den flanschseitigen Fluidtransport dar. Die zweite Vertiefungsart 22 hingegen dient zum Transport eines Fluids von einer Seite der Ventilscheibe zur anderen, so dass diese Vertiefung 22 stets mit einem Kanal 24 durch die Ventilscheibe hindurch in Verbindung steht und eine Vertiefung für einen Fluidtransport durch die Ventilscheibe darstellt. Der eingelassene Kanal innerhalb der Oberfläche der Ventilscheibe 20 ist hierbei typischerweise anders ausgeformt als bei der ersten Vertiefungsart 21.

Dabei können auf einer Seite der Ventilscheibe je nach Ausführungsform der Kolbenpumpe jeweils eine oder beide Vertiefungsarten vorgesehen sein. In der Ausführungsform der Figuren 2a und 2b kann erneut für beide Winkelpositionen eine einzelne Vertiefung auf jeder Seite der Ventilscheibe genutzt werden, da jede Seite nur eine Vertiefungsart erfordert.

Fig. 3a und 3b zeigen eine weitere Ausführungsform einer Kolbenpumpe 10, bei welcher am Flansch 15 ein zweiter Zylinder 11' und der Einlassstutzen 12 angebracht sind. An dem anderen Flansch 14 befinden sich der Auslassstutzen 13 und der erste Zylinder 11. Für diese Anordnung muss die Ventilscheibe 20 mit den verschiedenen Arten von Vertiefungen erneut modifiziert werden, um in den verschiedenen Winkelpositionen der Ventilscheibe 20 Fluid zwischen den beiden Seiten fördern zu können. Dabei ist auf jeder Seite der Ventilscheibe jeweils eine Vertiefung von jeder Art vorgesehen, so dass die rechte Seite eine Vertiefung 21 ohne Durchlasskanal und eine Vertiefung 22 mit Durchlasskanal 24 aufweist. Die linke Seite der Ventilscheibe weist ebenfalls eine Vertiefung 21' ohne Durchlasskanal und eine zweite Vertiefung 22' mit Durchlasskanal 24' auf. In dieser Ausführungsform können Vertiefungen auf einer Seite der Ventilscheibe somit nicht für zwei Winkelpositionen genutzt werden, da in den Winkelpositionen unterschiedliche Vertiefungsarten erfordert.

In der Kolbenstellung der Fig. 3a wird das Fluid aus der Kammer 23 durch die Zylinderöffnung 30 in die Vertiefung 21 und von dort durch den Durchlass 34 in den Auslassstutzen 13 gedrückt. Gleichzeitig wird Fluid aus dem Einlassstutzen 12 durch den Durchlass 35 in die Vertiefung 21' und von dort durch die Zylinderöffnung 33 in die Kammer 23' gesaugt. In dieser Winkelposition der Ventilscheibe 20 erfolgt somit kein Fluidtransport durch die Ventilscheibe 20 hindurch, sondern Fluid wird lediglich flanschseitig bewegt.

Bewegen sich die Kolben 16, 16' nun, wie in Fig. 3b dargestellt, in die andere Richtung, befindet sich die Ventilscheibe 20 in einer zweiten Winkelposition, so dass das zuvor in die Kammer 23' gesaugte Fluid nun durch die Zylinderöffnung 32 über die Vertiefung 22' und den Kanal 24' durch den Durchlass 34 in den Auslassstutzen 13 gedrückt wird. Gleichzeitig wird neues Fluid aus dem Einlassstutzen 12 durch den Durchlass 35 über den Kanal 24 in die Vertiefung 22 und von dort durch die Zylinderöffnung 31 in die Kammer 23 gesaugt. Auch so lässt sich eine nahezu unterbrechungsfreie Förderung erreichen, wobei in dieser Winkelposition ein ausschließlicher Fluidtransport durch die Ventilscheibe erfolgt.

Der prinzipielle Aufbau einer Ventilscheibe 20 ist in den Figuren 4a und 4b dargestellt, wobei es sich um eine Ausführungsform einer Mehrkolbenpumpe gemäß den Figuren 2a und 2b handelt und auf jeder Seite der Ventilscheibe 20 jeweils zwei Vertiefungen der gleichen Art vorgesehen sind. Blickrichtung in den beiden Abbildungen ist vom Zylinder 11' aus auf die Flansche 14, 15 entlang der Zylinderachse 26. Zylinder 11, 11', Stutzen 12, 13 und der Ventilscheibenumfang 25 werden so dargestellt, wie sie aus dieser Blickrichtung gesehen werden. Auf dem Flansch 15 in Fig. 4a ist die Projektion des Zylinders 11' als gestrichelter Kreis erkenntlich. Die Kreise 32 und 33 stellen die Zylinderöffnungen dieses Zylinders 11' dar und sind als Durchbrüche im Flansch 15 ausgeführt. Die Ventilscheibenfunktionen sind durch die Vertiefungen 22, 22' sowie durch zwei Durchlasskanäle 24, 24' zur gegenüber liegenden Ventilscheibenseite realisiert. Da die Zylinderöffnungen 32 und 33 in der gezeigten Ventilscheibenstellung von den Vertiefungen 22, 22' getrennt sind, ist der Zylinder 11' vollständig verschlossen.

Fig. 4b zeigt den Flansch 14 mit den Projektionen des Zylinders 11, sowie den Ein- und Auslassstutzen 12, 13 einer Pumpe, wobei diese Projektionen wieder als gestrichelte Kreise dargestellt sind. Die Zylinderöffnungen 30 und 31 des Zylinders 11 sind als Durchbrüche im Flansch 14 ausgeführt, während die Durchlässe in den Stutzen 12, 13 durch die Kreise 34 und 35 gekennzeichnet sind. Funktional bedingt weist diese Ventilscheibenseite weitere und deutlich längere Vertiefungen 21 und 21' auf, da über die Durchlässe 34, 35 im Flansch 14 Verbindungen zum Ein- und Auslassstutzen 12, 13 hergestellt werden müssen. Ähnlich den Öffnungen 30, 31 zum Zylinder 11 sind die Durchlässe 34, 35 als Flanschdurchbrüche ausgeführt. Bei der skizzierten Ventilscheibenstellung ist der Zylinder 11 ebenfalls durch die Ventilscheibe 20 verschlossen und vollständig vom Einlass- und Auslassstutzen 12, 13 abgetrennt.

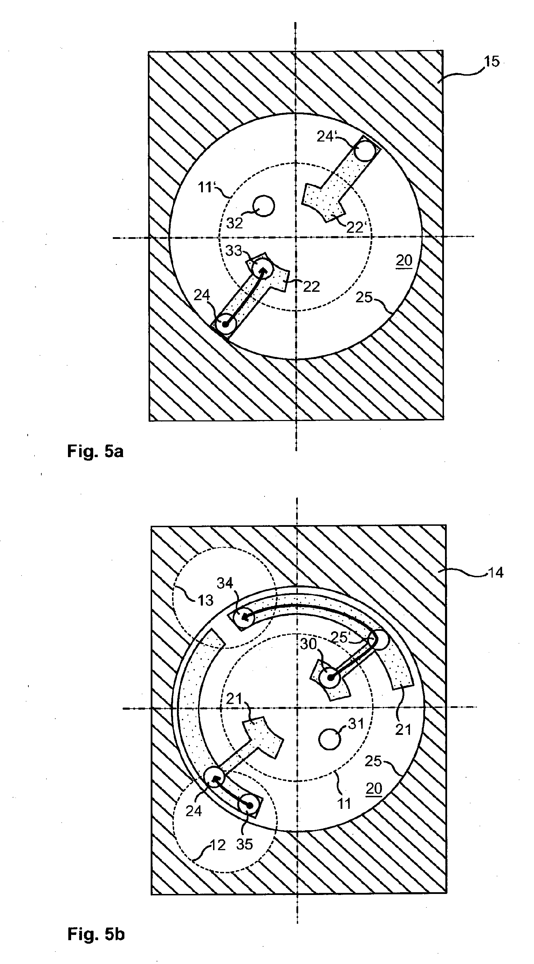

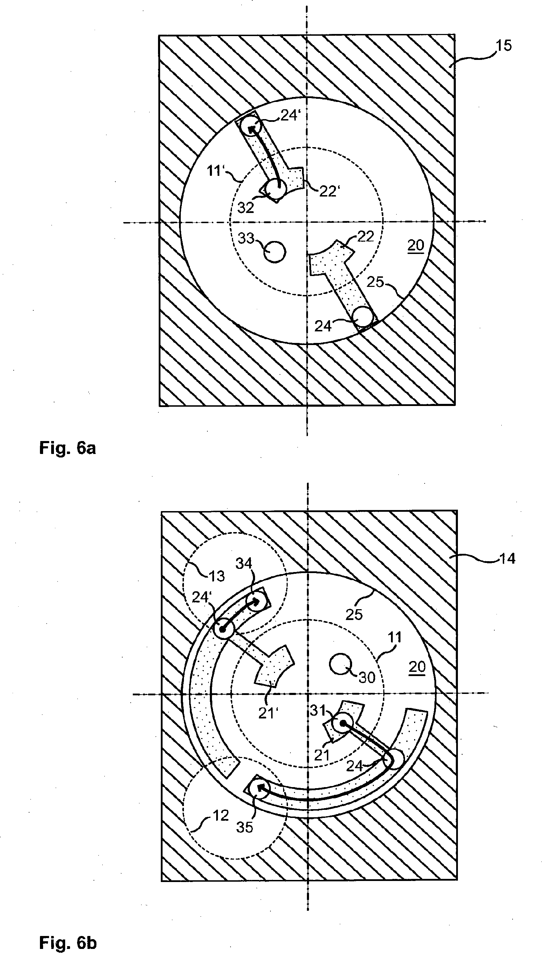

Der Funktionsmechanismus der Ventilscheibe 20 ist in den Figuren 5a bis 6b dargestellt, wobei die Ventilscheibe aus der Position der Figuren 4a und 4b herausbewegt wurde. Die Darstellungen sind analog in den Figuren 4a und 4b auf die beiden Flansche 14 und 15 bezogen. In Fig. 5a saugt der Zylinder 11' an und gleichzeitig stößt der Zylinder 11 aus, wie es in Fig. 5b gezeigt ist. Umgekehrt stößt Zylinder 11' in Fig. 6a aus, und Zylinder 11 saugt in Fig. 6b an.

Die in Fig. 5a dargestellte Drehung der Ventilscheibe 20 im Uhrzeigersinn führt dazu, dass die Vertiefung 22 die Einlassöffnung 33 des Zylinders 11' erreicht, während sich die gegenüber liegende Vertiefung 22' weiter von der Zylinderöffnung 32 entfernt. Die Vertiefungen auf der anderen Seite der Ventilscheibe drehen sich entsprechend ebenfalls, wobei durch den Durchlasskanal 24 die Vertiefung 21 auf der anderen Ventilscheibenseite erreicht wird. Diese Vertiefung 21 stellt über den Durchlass 35 eine Verbindung zum Einlassstutzen 12 dar, so dass der Zylinder 11' das Fluid aus dem Einlassstutzen 12 ansaugen kann. Dieser Fluidweg ist in den Figuren 5a und 5b durch Pfeile dargestellt.

Gleichzeitig ist die Öffnung 30 des Zylinders 11 in Fig. 5b über die Vertiefung 21' und den Durchlass 34 mit dem Auslassstutzen 13 verbunden. Zylinder 11 stößt somit das Fluid über den Auslassstutzen 13 aus, was in Fig. 5b ebenfalls durch einen Pfeil gezeigt ist und insgesamt der Situation in Fig. 2a entspricht.

In den Figuren 6a und 6b ist die Ventilscheibe 20 weiter im Uhrzeigersinn bzw. zurück gegen den Uhrzeigersinn gedreht. In der Darstellung der Fig. 6a ist dadurch nun die Vertiefung 22' mit der Öffnung 32 des Zylinders 11' verbunden. Über den Kanal 24' gelangt das zuvor angesaugte Fluid aus dem Zylinder 11' auf die andere Seite der Ventilscheibe 20 und gelangt von dort über den Durchlass 34 im Flansch 14 zum Auslassstutzen 13. Zum gleichen Zeitpunkt verbindet die Vertiefung 21 den Einlassstutzen 12 über den Durchlass 35 im Flansch 14 mit der Öffnung 31 des Zylinders 11. Somit ist die Ansaugung durch den Kolben 16 in Zylinder 11 gewährleistet, was insgesamt der Situation in Fig. 2b entspricht.

Die gewählte geometrische Anordnung der Zylinderöffnungen 30, 31, 32, 33, sowie die Ausführungsformen der Vertiefungen 21, 21', 22, 22' und der Durchlasskanäle 24, 24' bestimmen die winkelabhängige Ventilfunktion und insbesondere die Totzeit, d.h. der Winkelbereich, in dem die Ventilfunktion von Ansaugen auf Ausstoßen umgeschaltet wird und innerhalb derer keine Kolbenbewegung stattfinden darf.

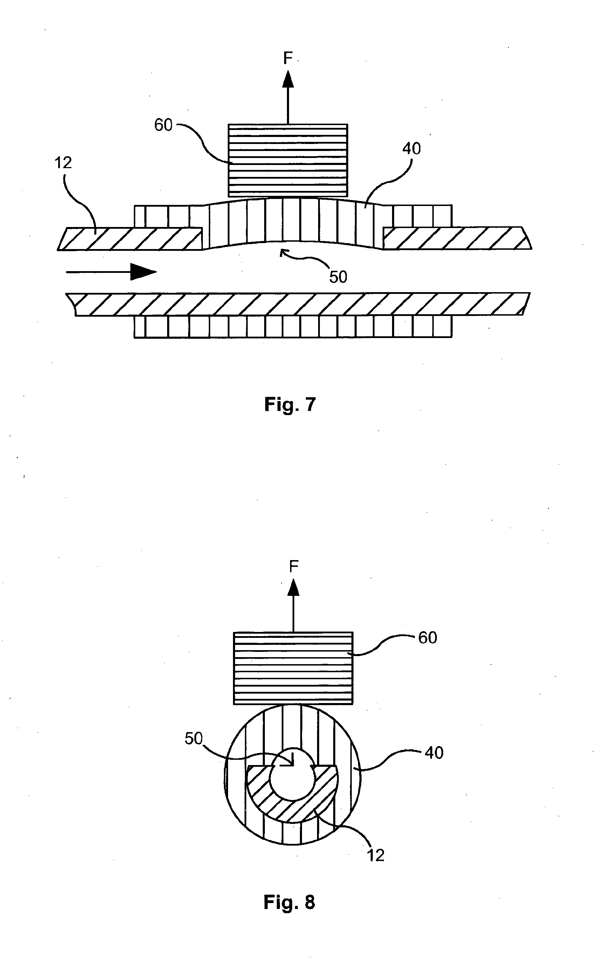

In den Einlass- und Auslassstutzen 12, 13 ist in den Ausführungsbeispielen der Figuren 1a bis 3b jeweils eine Sensorkomponente 40, 41 eingebracht, die anhand der Figuren 7 bis 12 näher erläutert werden soll. Dabei ist in den Figuren beispielhaft der betreffende Bereich in einem Einlassstutzen 12 mit einer Sensorkomponente 40 dargestellt.

Der in Fig. 7 dargestellte Längsschnitt durch einen Stutzen 12 zeigt eine außen liegende Sensorkomponente 40, welche den Stutzen 12 im Bereich einer Aussparung 50 formschlüssig umgibt. Hierbei wird eine dichte Verbindung zwischen dem Stutzen 12 und der schlauchförmigen Sensorkomponente 40 erreicht. Die Sensorkomponente 40 kann dabei an ihrer Innenseite so ausgeformt sein, dass sie sich teilweise in die Aussparung 50 einfügt, wie es in Fig. 7 dargestellt ist. Die Okklusionssensorik kann dabei vorteilhafterweise in einem spritzgusstechnischen Zweikomponentenprozess gefertigt werden, wobei die Sensorkomponente 40 als zweiter Prozessschritt nach der Herstellung eines rohrförmigen Stutzens 12 durch eine Hartkomponente aufgebracht wird. Als Material für die Hartkomponente kann ein Hartkunststoff gewählt werden, während die Sensorkomponente aus einem elastischen und druckempfindlichen Material wie einem Elastomer gefertigt wird.

Die Aussparung 50 kann einen beliebigen Querschnitt haben, wobei sich runde Querschnitte für eine gleichmäßige Kraftverteilung als vorteilhaft erwiesen haben. Ferner sollte die Größe der Aussparung 50 geeignet gewählt werden. In Fig. 8 ist beispielsweise ein Querschnitt durch die Mitte des Längsschnitts aus Fig. 7 gezeigt, bei dem die Aussparung 50 sehr tief gewählt wurde und annähernd bis zur Mittellinie des Stutzens 12 reicht.

Durch die Aussparung 50 kann dann ein Kraftsensor 60 reichen, um in diesem Bereich Kontakt mit der Außenseite der Sensorkomponente 40 herzustellen und die Verformung der Membran 20 mechanisch abzugreifen. Dies kann beispielsweise über einen Stempel 60 erfolgen, der an der Sensorkomponente 40 anliegt. Erhöht sich der Innendruck im Stutzen 12 aufgrund einer Okklusion, wölbt sich die Sensorkomponente 40 nach außen, was von dem Stempel 60 detektiert werden kann. Reduziert sich der Druck im Stutzen 12 aufgrund einer Okklusion, verringert sich die Wölbung der Sensorkomponente 41, was ebenfalls durch den Stempel 60 detektiert werden kann.

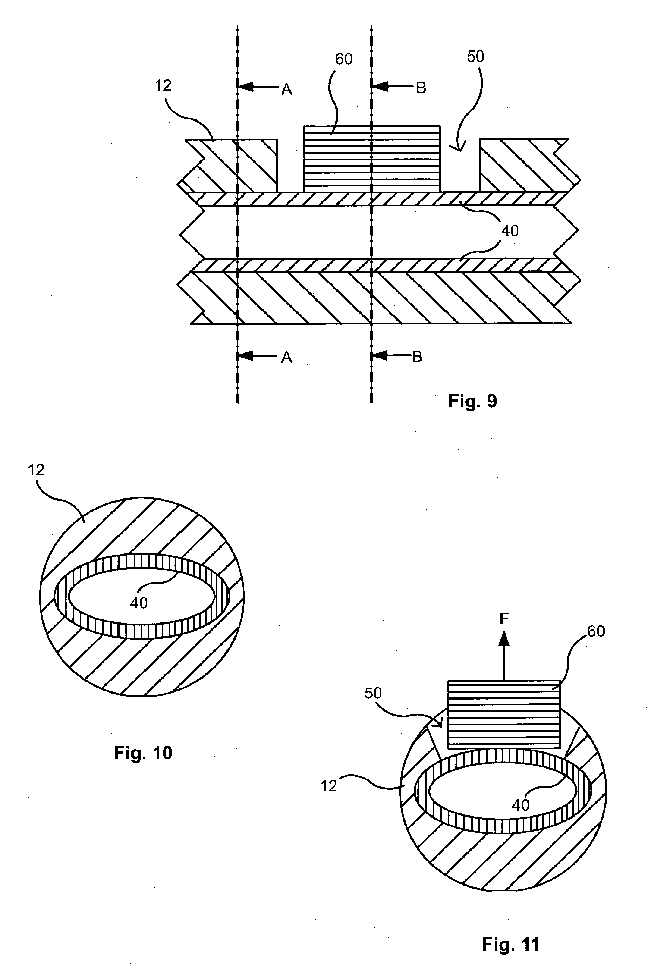

Fig. 9 zeigt ein zweites Ausführungsbeispiel der Erfindung, bei dem eine schlauchförmige Sensorkomponente 40 innerhalb eines Stutzens 12 angebracht ist und somit von innen eine Aussparung 50 dicht abdeckt. Diese Okklusionssensorik kann hierbei ebenfalls in einem spritzgusstechnischen Zweikomponentenprozess in Form eines durchgehenden Innenschlauchs als Weichkomponente hergestellt werden, während der zugehörige Einlass- oder Auslassstutzen als darüber liegendes Stützrohr bzw. Stützskelett als Hartkomponente integral und nicht zerlegbar gefertigt wird. Dabei kann die Innenfläche des Stutzens 12 auch so ausgebildet sein, dass sie den Schlauch 40 in seiner Position hält und an einem axialen Verrutschen hindert (nicht dargestellt).

Fig. 10 zeigt einen ersten Querschnitt durch einen solchen Stutzen entlang der Linie A-A, wodurch ersichtlich wird, dass die Sensorkomponente 40 einen elliptischen Querschnitt hat. Die Innenwandung des Stutzens 12 ist entsprechend ausgeformt, um die Sensorkomponente 40 formschlüssig aufnehmen zu können. Ein zweiter Querschnitt entlang der Linie B-B ist in Fig. 11 dargestellt und zeigt auch den Stempel 60, der durch die Aussparung 50 hindurch die Außenfläche der Sensorkomponente kontaktiert.

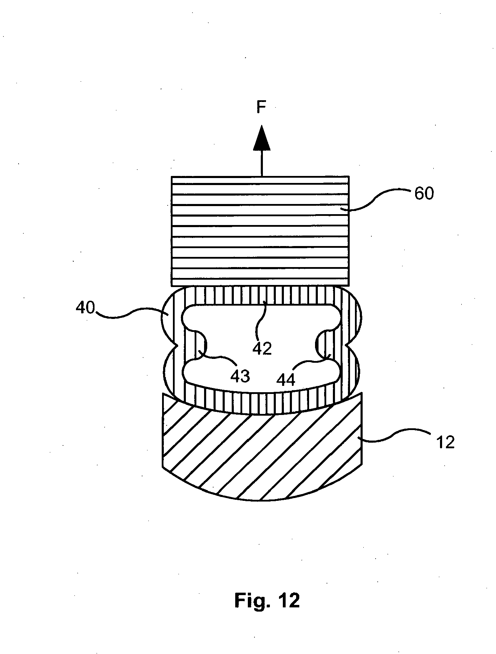

Um Eigenspannungen der Sensorkomponente 40 weitestgehend zu verhindern, kann diese auch als speziell geformte Messmembran ausgebildet sein, wie sie beispielsweise in Fig. 12 dargestellt ist. Hierbei weist die Messmembran 40 zwei gegenüber liegende Membranseiten 43 und 44 auf, die nach innen geknickt sind. Die Membranoberseite 42, welche die beiden Membranseiten 43, 44 verbindet, ist gerade ausgeführt und steht in Kontakt mit dem Stempel 60. Die Membranoberseite 42 verändert sich so nicht mehr durch die Eigenspannung, was eine lineare Kraftkennlinie Kraft = Innendruck x Membranfläche ergibt.

Der Querschnitt der Sensorkomponente 40 ist somit individuell gestaltet und enthält wenigstens eine der folgenden funktionalen Komponenten:
□ Eine gerade oder einer Geraden ähnliche Linie, wehe die Geometrie der für die Messzwecke benötigte Membran bestimmt
□ Eine gerade oder gebogene Linie gegenüber der Membran, die eine Stützfunktion der Weichkomponente gegenüber der rohr- oder skelettförmigen Hartkomponente wahrnimmt.
□ Eine Geometrie zur Realisierung einer federnden Funktion an den beiden Seiten der Weichkomponente, damit eine Vorspannung aufgebaut werden kann, die zur Messung von Drücken unterhalb des atmosphärischen Umgebungsdrucks notwendig ist. Darüber hinaus ist die federnde Funktion notwendig, damit sich die Membran bei einem steigenden Innendruck von ihrer gegenüberliegenden Stützfläche entfernen kann.

Die Innenfläche des Stutzens 12 kann dann entsprechend ausgeführt sein, so dass die Messmembran 40 formschlüssig an diesem anliegt und sich bei einer Druckerhöhung nicht in unerwünschte Richtungen z.B. zur Seite hin ausdehnt. Diese spezielle Ausformung des Stutzens 12 kann auch nur im Bereich der Okklusionssensorik gegeben sein, wodurch sich aufwändige Formen innerhalb des gesamten Stutzens vermeiden lassen.

Die Hartkomponente, die den Stempel 60 umgibt, weist vorzugsweise eine ebene Fläche auf, die etwas unterhalb der Stempeloberkante liegt. Diese Fläche dient als Anschlagsfläche, wenn der Stempel gegen eine andere Fläche gedrückt wird. Der Stempel kann dann nur um den Betrag seines Überstandes eingedrückt werden, wodurch eine konstante Vorspannung für die Drucksensorik erzeugt wird.

Bei den in den Figuren 7 bis 12 dargestellten Ausführungsbeispielen befindet sich die Aussparung 50 und somit der Stempel 60 im Stutzen 12 stets oben, es können jedoch auch andere Anordnungen gewählt werden.

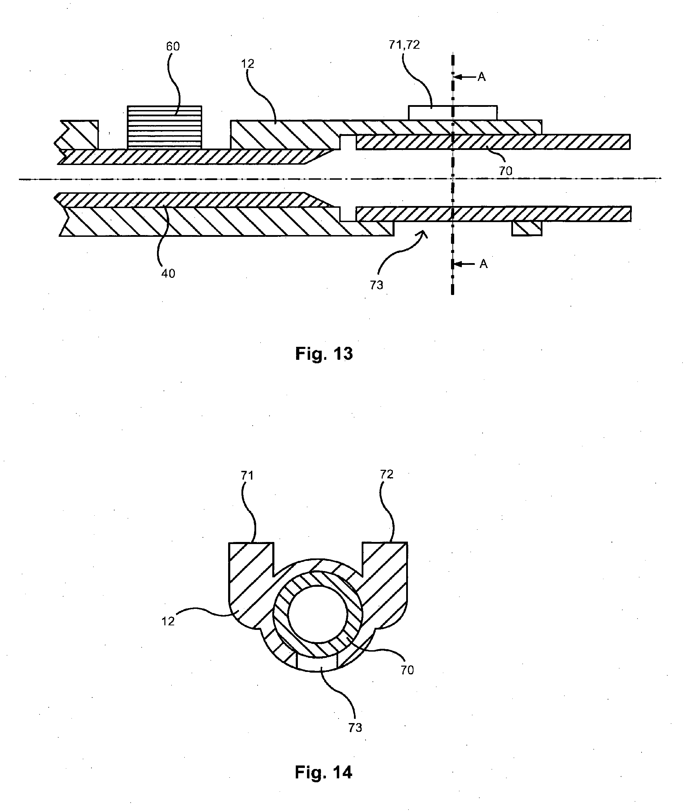

In Fig. 13 ist zusammen mit einer innen liegenden Sensorkomponente 40 eine optionale Ultraschallsensorik für die erfindungsgemäße Kolbenpumpe 10 gezeigt, die das zusätzliche manuelle Einführen eines Schlauchs in spezielle Halterungen überflüssig macht. Mittels dieser Ultraschallsensorik können Luftblasen im Infusionsschlauchsystem erkannt werden, wobei auch diese Ultraschallsensorik im Einlass- und/oder Auslassstutzen 12, 13 angebracht werden kann. In Fig. 13 ist die Ultraschallsensorik beispielhaft im Einlassstutzen 12 dargestellt. Hierzu ist dieser Stutzen 12 an seinem Ende innen entsprechend aufgeweitet, so dass dort ein flexibler Schlauch 70 eingeführt und dort beispielsweise durch Kleben fixiert werden kann. Die Ein- und Auskopplung des Ultraschalls erfolgt an zwei gegenüberliegenden Flächen 71, 72, die vorzugsweise plan ausgeführt sein können, wie es in Fig. 14 im Querschnitt entlang der Linie A-A gezeigt ist. Die beiden Flächen 71, 72 liegen dabei in einer Ebene. Es sind jedoch auch andere formschlüssige Verbindungen wie z.B. über einen Konus denkbar. Gegenüber den planen Flächen 71, 72 befindet sich eine Aussparung 73 im Einlassstutzen 12.

Wie die Okklusionssensorik bilden die mechanischen Komponenten für die Luftblasenerkennung vorzugsweise ebenfalls einen integralen Bestandteil der rohrförmigen Stutzen und können nicht zerstörungsfrei demontiert werden. Vergleichbare Anpassungen der Pumpvorrichtung zur Unterstützung der Sensorik sind ebenfalls möglich, um beispielsweise alternative optische Luftblasenerkennungsmethoden oder die Bildung von definierten Schnittstellen für eine Temperaturmessung zu ermöglichen.

Die Ein- und Auskoppelflächen für den Ultraschall sowie die Anschlagsfläche für die Okklusionssensorik bilden vorzugsweise eine Ebene, wodurch die Schnittstelle zu den zugehörigen elektronischen Sensoren ebenfalls eine Ebene bildet, die sich z.B. in einem medizinischen Gerät befinden kann. Durch diese Maßnahme lassen sich Anforderungen an eine gute und einfache Reinigungsmöglichkeit vorteilhaft umsetzen.

### Bezugszeichenliste

- 10: Kolbenpumpe, Einkolbenpumpe, Mehrkolbenpumpe
- 11,11': Zylinder
- 12: Stutzen, Einlassstutzen
- 13: Stutzen, Auslassstutzen
- 14,15: Flansch, Montageflasch
- 16,16': Kolben
- 20: Ventilscheibe
- 21,21': Fluidüberführungsmittel, Vertiefung für flanschseitigen Fluidtransport
- 22,22': Fluidüberführungsmittel, Vertiefung für Fluidtransport durch Ventilscheibe
- 23,23': Kammer
- 24,24': Fluidüberführungsmittel, Durchlasskanal
- 25: Umfangsfläche der Ventilscheibe
- 26: Drehachse der Ventilscheibe, Zylinderachse
- 30,31,32,33: Zylinderöffnung
- 34,35: Durchlass in Stutzen
- 40,41: Sensorkomponente, Messmembran
- 42: Membranoberseite
- 43,44: Membranseite
- 50: Aussparung für Okklusionssensorik
- 60: Kraftsensor, Stempel
- 70: Schlauchstück
- 71,72: Ein- und Auskoppelungsfläche
- 73: Aussparung für Ultraschallsensorik

## Patentansprüche

1. Kolbenpumpe (10) zur Förderung eines Fluids,
umfassend wenigstens einen Zylinder (11; 11') mit einem Kolben (16; 16'), der mittels eines Antriebs innerhalb des Zylinders (11; 11') entlang der Längsachse des Zylinders (11; 11') bewegbar ist,
wobei jeder Zylinder (11; 11') an einer Stirnseite einen Montageflansch (14; 15) mit wenigstens einer Zylinderöffnung (30; 31; 32; 33) aufweist und sich zylinderseitig zwischen jedem Kolben (16; 16 ') und dem Montageflansch (14; 15) eine Kammer (23; 23') mit veränderlichem Volumen bildet, wenn der zugehörige Kolben (16; 16') im Zylinder (11; 11') bewegt wird,
und die Kolbenpumpe (10) einen Einlassstutzen (12) zur Zuführung und einen Auslassstutzen (13) zur Abführung des Fluids aufweist, wobei auf der einem Zylinder (11; 11') abgewandten Seite des Montageflansches (14; 15) eine drehbare Ventilscheibe (20) angeordnet ist,
die an dem jeweiligen Montageflansch (14; 15) anliegt,
und dass an dem wenigstens einen Montageflansch (14; 15) der Einlassstutzen (12) und/oder Auslassstutzen (13) angebracht ist,
wobei der Montageflansch (14; 15) im Bereich eines Einlassstutzens (12) und eines Auslassstutzens (13) jeweils wenigstens einen Durchlass (34; 35) aufweist, durch den das Fluid zwischen dem Einlassstutzen (12) und/oder dem Auslassstutzen (13) und der anderen Seite des Montageflansches (14; 15) strömen kann,
**dadurch gekennzeichnet, dass** die Ventilscheibe (20) an wenigstens einer Oberfläche eine zur abgewandten Oberfläche hin verschlossene, eingelassene Vertiefung (21; 21') als Fluidüberführungsmittel aufweist, durch welche bei Drehung der Ventilscheibe (20) zwischen zwei Winkelpositionen in beiden Winkelpositionen jeweils wenigstens eine Zylinderöffnung (30; 31; 32; 33) mit einem Durchlass (34; 35) eines Einlass- und/oder Auslassstutzens (12; 13) verbindbar ist.

2. Kolbenpumpe nach Anspruch 1,
**dadurch gekennzeichnet, dass** die Fluidüberführungsmittel der Ventilscheibe (20) bei Drehung der Ventilscheibe (20) in wenigstens einer dritten Winkelposition keine Zylinderöffnung (30; 31; 32; 33) mit einem Durchlass (34; 35) eines Einlass- und/oder Auslassstutzens (12; 13) verbinden.

3. Kolbenpumpe nach einem der Ansprüche 1 und 2,
**dadurch gekennzeichnet, dass** jeder Einlassstutzen und/oder Auslassstutzen (12; 13) in Richtung des wenigstens einen Zylinders (11; 11') verläuft.

4. Kolbenpumpe nach einem der Ansprüche 1 bis 3,
**dadurch gekennzeichnet, dass** zwei Montageflansche (14; 15) vorgesehen sind, zwischen denen sich die Ventilscheibe (20) befindet,
wobei an beiden Montageflanschen (14; 15) jeweils ein Zylinder (11; 11') mit wenigstens einer Zylinderöffnung (30; 31; 32; 33) angebracht ist,
während sich die Einlass- und Auslassstutzen (12; 13) gemeinsam an einem der beiden Montageflansche (14) befinden.

5. Kolbenpumpe nach einem der Ansprüche 1 bis 3,
**dadurch gekennzeichnet, dass** zwei Montageflansche (14; 15) vorgesehen sind, zwischen denen sich die Ventilscheibe (20) befindet, wobei an beiden Montageflanschen (14; 15) jeweils ein Zylinder (11; 11') mit wenigstens einer Zylinderöffnung (30; 31; 32; 33) und jeweils einer der Einlass- oder Auslassstutzen (12; 13) angebracht sind.

6. Kolbenpumpe (10) nach einem der Ansprüche 1 bis 5,
**dadurch gekennzeichnet, dass** die wenigstens eine Vertiefung (21; 21') in eine einem Montageflansch (14; 15) mit Einlass- und/oder Auslassstutzen (12; 13) zugewandten Fläche der Ventilscheibe (20) eingelassen ist,
wobei die Vertiefung (21; 21') so ausgebildet ist, dass sie durch Drehung der Ventilscheibe (20) gleichzeitig mit jeweils einer Zylinderöffnung (30; 31; 32; 33) und einem Durchlass (34; 35) eines Einlass- und/oder Auslassstutzens (12; 13) in Deckung bringbar ist.

7. Kolbenpumpe (10) nach einem der Ansprüche 1 bis 6,
**dadurch gekennzeichnet, dass** die wenigstens eine Vertiefung (22; 22') in eine einem Montageflansch (14; 15) mit Einlass- und/oder Auslassstutzen (12; 13) zugewandten Fläche der Ventilscheibe (20) eingelassen ist
und ferner einen Durchlasskanal (24; 24) aufweist, der sich von der Vertiefung (22; 22') aus durch die Ventilscheibe (20) hindurch erstreckt, wobei die Vertiefung (22; 22') und der zugehörige Durchlasskanal (24; 24') so ausgebildet sind, dass sie durch Drehung der Ventilscheibe gleichzeitig mit jeweils einer Zylinderöffnung (30; 31; 32; 33) und dem Durchlass (34; 35) eines gegenüber liegenden Einlass- und/oder Auslassstutzens (12; 13) in Deckung bringbar sind.

8. Kolbenpumpe nach einem der Ansprüche 1 bis 7,
**dadurch gekennzeichnet, dass** in einen Einlassstutzen (12) und/oder in einen Auslassstutzen (13) jeweils eine Okklusionssensorik integriert ist, die nicht zerstörungsfrei demontierbar ist.

9. Kolbenpumpe nach Anspruch 8,
**dadurch gekennzeichnet, dass** in dem Einlass- und/oder Auslassstutzen (12; 13) wenigstens eine Aussparung (50) vorgesehen ist, welche dicht durch eine Sensorkomponente (40; 41) abgedeckt wird, die aus einem druckempfindlichen Material besteht,
wobei das Material des Einlass- und/oder Auslassstutzens (12; 13) härter ist als das der Sensorkomponente (40; 41),
und dass die Kolbenpumpe (10) einen Kraftsensor (60) aufweist, mit dem druckinduzierte Veränderungen der Sensorkomponente (40; 41) im Bereich der jeweiligen Aussparung (50) messbar sind.

10. Kolbenpumpe nach Anspruch 9,
**dadurch gekennzeichnet, dass** die Verbindung zwischen der Sensorkomponente (40) und einem Einlass- und/oder Auslassstutzen (12; 13) eine Spritzgussverbindung ist, die durch ein Zweikomponentenverfahren hergestellt wurde.

11. Kolbenpumpe nach einem der Ansprüche 9 und 10,
**dadurch gekennzeichnet, dass** die Sensorkomponente (40; 41) schlauchförmig ist und so an dem Einlass- und/oder Auslassstutzen (12; 13) angebracht ist, dass sie die jeweilige Aussparung (50) von innen oder von außen dicht abdeckt.

12. Kolbenpumpe nach einem der Ansprüche 1 bis 11,
**dadurch gekennzeichnet, dass** in einen Einlassstutzen (12) und/oder in einen Auslassstutzen (13) jeweils eine Ultraschallsensorik zur Erkennung von Luftblasen im jeweiligen Stutzen (12; 13) integriert ist, die nicht zerstörungsfrei demontierbar ist.

13. Kolbenpumpe nach Anspruch 12,
**dadurch gekennzeichnet, dass** in den Einlass- und/oder Auslassstutzen (12; 13) formschlüssig ein Schlauch (70) eingesteckt ist, durch den Fluid dem Einlassstutzen (12) zugeführt oder durch den Fluid aus dem Auslassstutzen (13) abgeführt wird, wobei im Bereich des Schlauches (70) an zwei Seiten des betreffenden Einlass- und/oder Auslassstutzens (12; 13) Flächen (71; 72) zur Ein- und Auskopplung von Ultraschall vorgesehen sind.

14. Kolbenpumpe nach Anspruch 13,
**dadurch gekennzeichnet, dass** der Einlass- und/oder Auslassstutzen (12; 13) so ausgeformt ist, dass die Flächen (71; 72) in einer Ebene liegen.

15. Kolbenpumpe nach Anspruch 14,
**dadurch gekennzeichnet, dass** der Einlass- und/oder Auslassstutzen (12; 13) wenigstens eine weitere Aussparung (73) aufweist.

## Claims

1. Piston pump (10) for pumping a fluid,
including at least one cylinder (11; 11') having a piston (16; 16') which is movable inside the cylinder (11; 11') along the longitudinal axis of the cylinder (11; 11') by means of a drive,
wherein each cylinder (11; 11') comprises at an end face a mounting flange (14; 15) having at least one cylinder opening (30; 31; 32; 33)
and forms on the cylinder-side, between each piston (16; 16') and the mounting flange (14; 15), a chamber (23; 23') having a volume that changes when the associated piston (16; 16') is moved in the cylinder (11; 11'),
and the piston pump (10) has an inlet port (12) for supplying and an outlet port (13) for draining the fluid, wherein
a rotatable valve plate (20) is arranged on the side of the mounting flange (14; 15) which side faces away from a cylinder (11; 11'),
the valve plate (20) bearing on the respective mounting flange (14; 15) and that is attached to the at least one mounting flange (14; 15) of the inlet port (12) and/or outlet port (13),
wherein the mounting flange (14; 15) has at least one passage (34; 35) in the region of an inlet port (12) and an outlet port (13), respectively, wherein through this passage (34; 35), the fluid can flow between the inlet port (12) and/or the outlet port (13) and the other side of the mounting flange (14; 15), **characterized in that** the valve plate (20) has on at least one surface a recessed cavity (21; 21') as fluid-transporting means, which is blocked in the direction of the averted surface, by which, upon rotation of the valve plate (20) between two angular positions, in each of the two angular positions at least one cylinder opening (30; 31; 32; 33) can be connected to a passage (34; 35) of an inlet and/or outlet port (12; 13).

2. Piston pump according to claim 1,
**characterized in that**, upon rotation of the valve plate (20) to at least a third position, the fluid-transporting means of the valve plate (20) do not connect a cylinder opening (30; 31; 32; 33) with a passage (34; 35) of an inlet and/or outlet port (12; 13).

3. Piston pump according to one of claims 1 and 2,
**characterized in that** each inlet port and/or outlet port (12; 13) runs in the direction of the at least one cylinder (11; 11').

4. Piston pump according to one of claims 1 to 3,
**characterized in that** two mounting flanges (14; 15) are provided, between which the valve plate (20) is located,
wherein a respective cylinder (11; 11') having at least one cylinder opening (30; 31; 32; 33) is attached to each of the two mounting flanges (14; 15),
while the inlet and outlet ports (12: 13) are located together at one of the two mounting flanges (14).

5. Piston pump according to one of claims 1 to 3,
**characterized in that** two mounting flanges (14; 15) are provided between which the valve plate (20) is located, wherein a respective cylinder (11; 11') having at least one cylinder opening (30; 31; 32; 33), and a respective one of the inlet or outlet ports (12: 13) is attached to both mounting flanges (14; 15) respectively.

6. Piston pump (10) according to one of claims 1 to 5,
**characterized in that** the at least one cavity (21; 21') is recessed into a surface of the valve plate (20), said surface facing a mounting flange (14; 15) having inlet and/or outlet port (12; 13),
wherein the cavity (21; 21') is configured such that by rotating the valve plate (20) it can be made to coincide simultaneously with a cylinder opening (30; 31; 32; 33) and a passage (34; 35) of an inlet and/or outlet port (12; 13) respectively.

7. Piston pump (10) according to one of claims 1 to 6,
**characterized in that** the at least one cavity (22; 22') is recessed into a surface of the valve plate (20), said surface facing a mounting flange (14; 15) having an inlet and/or outlet port (12; 13)
and furthermore comprises a passage channel (24; 24) which extends out from the cavity (22; 22') through the valve plate (20), wherein the cavity (22; 22') and the associated passage channel (24; 24') are configured such that by rotating the valve plate they can be made to coincide simultaneously with a cylinder opening (30; 31; 32; 33) and the passage (34; 35) of an opposite-lying inlet and/or outlet port (12; 13) respectively.

8. Piston pump (10) according to one of claims 1 to 7,
**characterized in that** an occlusion sensor is integrated into an inlet port (12) and/or outlet port (13) respectively, wherein the occlusion sensor cannot be non-destructively disassembled.

9. Piston pump according to claim 8,
**characterized in that** at least one recess (50) is provided in the inlet and/or outlet port (12; 13), which is tightly covered by a sensor component (40; 41) composed of a pressure sensitive material,
wherein the material of the inlet and/or outlet port (12; 13) is harder than that of the sensor component (40; 41),
and that the piston pump (10) has a force sensor (60) with which pressure-induced changes of the sensor component (40; 41) in the region of the recess can be measured.

10. Piston pump according to claim 9,
**characterized in that** the connection between the sensor component (40) and an inlet and/or outlet port (12; 13) is an injection-molded connection made by a two-component process.

11. Piston pump according to one of claims 9 and 10,
**characterized in that** the sensor component (40; 41) is tubular and is attached to the inlet and/or outlet port (12; 13) such that it tightly covers the respective recess (50) from inside or from outside.

12. Piston pump according to one of claims 1 to 11,
**characterized in that** an ultrasound sensor is integrated into an inlet port (12) and/or outlet port (13) respectively, for the detection of air bubbles in the respective port (12; 13), wherein the ultrasound sensor cannot be non-destructively disassembled.

13. Piston pump according to claim 12,
**characterized in that** a tube (70) is inserted into the inlet and/or outlet port (12; 13) with form-locking fit, through which fluid is supplied to the inlet port (12) or discharged from the outlet port (13), wherein surfaces (71; 72) for the coupling and decoupling of ultrasound are provided in the region of the tube (70) on two sides of the relevant inlet and/or outlet port (12; 13).

14. Piston pump according to claim 13,
**characterized in that** the inlet and/or outlet port (12; 13) is formed such that the surfaces (71; 72) lie in a plane.

15. Piston pump according to claim 14,
**characterized in that** the inlet and/or outlet port (12; 13) comprises at least one further recess (73).

## Revendications

1. Pompe à pistons (10) pour le refoulement d'un fluide,
comprenant au moins un cylindre (11 ; 11') avec un piston (16 ; 16'), qui peut être déplacé à l'aide d'un entraînement à l'intérieur du cylindre (11 ; 11') le long de l'axe longitudinal du cylindre (11 ; 11'),
chaque cylindre (11 ; 11') comprenant, au niveau d'un côté frontal, une bride de montage (14 ; 15) avec au moins une ouverture de cylindre (30 ; 31 ; 32 ; 33) et formant, coté cylindre, entre chaque piston (16 ; 16') et la bride de montage (14 ; 15), une chambre (23 ; 23') à volume variable, lorsque le piston (16 ; 16') correspondant est déplacé dans le cylindre (11 ; 11'),
et la pompe à pistons (10) comprenant un manchon d'entrée (12) pour l'alimentation et un manchon de sortie (13) pour l'évacuation du fluide,
un disque de soupape rotatif (20) étant disposé sur le côté de la bride de montage (14 ; 15) opposé à un cylindre (11 ; 11'),
qui s'appuie contre la bride de montage (14 ; 15) correspondante,
et le manchon d'entrée (12) et/ou le manchon de sortie (13) étant disposé au niveau de l'au moins une bride de montage (14 ; 15),
la bride de montage (14 ; 15) comprenant, au niveau d'un manchon d'entrée (12) et d'un manchon de sortie (13), au moins un passage (34 ; 35) par lequel le fluide peut s'écouler entre le manchon d'entrée (12) et/ou le manchon de sortie (13) et l'autre côté de la bride de montage (14; 15),
**caractérisée en ce que** le disque de soupape (20) comprend, au niveau d'au moins une face supérieure, un creux (21 ; 21') intégré et fermé en direction de la face supérieure opposée, qui sert de moyen de transfert de fluide, par lequel, lors de la rotation du disque de soupape (20) entre deux positions angulaires, dans les deux positions angulaires, au moins une ouverture de cylindre (30 ; 31 ; 32 ; 33) peut être reliée à un passage (34 ; 35) d'un manchon d'entrée et/ou de sortie (12 ; 13).

2. Pompe à pistons selon la revendication 1, **caractérisée en ce que** les moyens de transfert de fluide du disque de soupape (20) ne relient, lors de la rotation du disque de soupape (20) dans au moins une troisième position angulaire, aucune ouverture de cylindre (30 ; 31 ; 32 ; 33) avec un passage (34 ; 35) d'un manchon d'entrée et/ou de sortie (12 ; 13).

3. Pompe à pistons selon l'une des revendications 1 et 2, **caractérisée en ce que** chaque manchon d'entrée et/ou de sortie (12 ; 13) s'étend en direction de l'au moins un cylindre (11 ; 11').

4. Pompe à pistons selon l'une des revendications 1 à 3, **caractérisée en ce que** deux brides de montage (14 ; 15) sont prévues, entre lesquelles se trouve le disque de soupape (20),
un cylindre (11 ; 11') avec au moins une ouverture de cylindre (30 ; 31 ; 32 ; 33) étant monté sur chacune des deux brides de montage (14 ; 15),
tandis que les manchons d'entrée et de sortie (12 ; 13) se trouvent ensemble sur une des brides de montage (14).

5. Pompe à pistons selon l'une des revendications 1 à 3, **caractérisée en ce que** deux brides de montage (14 ; 15) sont prévues, entre lesquelles se trouve le disque de soupape (20), un cylindre (11 ; 11') avec au moins une ouverture de cylindre (30 ; 31 ; 32 ; 33) et un des manchons d'entrée ou de sortie (12 ; 13) étant montés sur chacune des deux brides de montage (14 ; 15).

6. Pompe à pistons (10) selon l'une des revendications 1 à 5, **caractérisée en ce que** l'au moins un creux (21 ; 21') est intégré dans une surface du disque de soupape (20) orientée vers une bride de montage (14 ; 15) avec manchon d'entrée et/ou de sortie (12 ; 13),
le creux (21 ; 21') étant conçu de façon à ce que, du fait de la rotation du disque de soupape (20), peut être amené simultanément en superposition avec une ouverture de cylindre (30 ; 31 ; 32 ; 33) et un passage (34 ; 35) d'un manchon d'entrée et/ou de sortie (12 ; 13).

7. Pompe à pistons (10) selon l'une des revendications 1 à 6, **caractérisée en ce que** l'au moins un creux (22 ; 22') est intégré dans une surface du disque de soupape (20) orientée vers une bride de montage (14 ; 15) avec manchon d'entrée et/ou de sortie (12 ; 13),
et comprend, en outre, un canal de passage (24 ; 24'), qui s'étend, à partir du creux (22 ; 22'), à travers le disque de soupape (20), le creux (22 ; 22') et le canal de passage (24 ; 24') correspondant étant conçus de façon à ce que, du fait de la rotation du disque de soupape, il puisse être amené simultanément en superposition avec une ouverture de cylindre (30 ; 31 ; 32 ; 33) et le passage (34 ; 35) d'un manchon d'entrée et/ou de sortie (12 ; 13) situé en face.

8. Pompe à pistons (10) selon l'une des revendications 1 à 7, **caractérisée en ce que**, dans chaque manchon d'entrée (12) et/ou dans un manchon de sortie (13) est intégré un système de capteurs d'occlusion qui peut être démonté de manière non destructible.

9. Pompe à pistons selon la revendication 8, **caractérisée en ce que**, dans le manchon d'entrée et/ou de sortie (12 ; 13), au moins un évidement (50) est prévu, qui est recouvert de manière étanche par un composant de capteur (40 ; 41) constitué d'un matériau sensible à la pression,
le matériau du manchon d'entrée et/ou de sortie (12 ; 13) étant plus dur que celui du composant de capteur (40 ; 41),
et **en ce que** la pompe à pistons (10) comprend un capteur de force (60) permettant de mesurer les modifications, dues à la pression, du composant de capteur (40 ; 41) au niveau de l'évidement (50) correspondant.

10. Pompe à pistons selon la revendication 9, **caractérisée en ce que** la liaison entre le composant de capteur (40) et un manchon d'entrée et/ou de sortie (12 ; 13) est une liaison moulée par injection fabriquée à l'aide d'un procédé à deux composants.

11. Pompe à pistons selon l'une des revendications 9 et 10, **caractérisée en ce que** le composant de capteur (40 ; 41) présente la forme d'une gaine et est monté sur le manchon d'entrée et/ou de sortie (12 ; 13) de façon à ce qu'il recouvre de l'intérieur ou de l'extérieur, de manière étanche, l'évidement (50) correspondant.

12. Pompe à pistons selon l'une des revendications 1 à 11, **caractérisée en ce que**, dans un manchon d'entrée (12) et/ou dans un manchon de sortie (13), est intégré un système de capteurs à ultrasons pour la détection de bulles d'air dans le manchon (12 ; 13) correspondant, qui n'est pas démontable de manière non destructible.

13. Pompe à pistons selon la revendication 12, **caractérisée en ce que**, dans le manchon d'entrée et/ou de sortie (12 ; 13), est inséré, par complémentarité de forme, un tuyau (70) à travers lequel un fluide est introduit dans le manchon d'entrée (12) ou à travers lequel un fluide est évacué par le manchon de sortie (13), des surfaces (71 ; 72) pour le couplage et le découplage des ultrasons étant prévues au niveau du tuyau (70) sur deux côtés du manchon d'entrée et/ou de sortie (12 ; 13).

14. Pompe à pistons selon la revendication 13, **caractérisée en ce que** le manchon d'entrée et/ou de sortie (12 ; 13) est formé de façon à ce que les surfaces (71 ; 72) se trouvent dans un plan.

15. Pompe à pistons selon la revendication 14, **caractérisée en ce que** le manchon d'entrée et/ou de sortie (12 ; 13) comprend au moins un évidement (73) supplémentaire.
